# EUROPEAN PATENT APPLICATION

(11) **EP 1 810 675 A1**
(43) Date of publication of application: **25.07.2007**
(21) Application number: 06290120.2
(22) Date of filing: 18.01.2006
(51) Int. Cl.: A61K 31/341, A61K 31/277, A61K 31/436, A61K 38/13, G01N 33/50, A61P 25/28

(54) **Method for treating Huntington's disease by inhibiting dephosphorylation of huntingtin at S421**

(71) Applicant: Institut Curie, 75248 Paris Cedex 05 (FR); CENTRE NATIONAL DE LA RECHERCHE SCIENTIFIQUE (CNRS), 75794 Paris Cedex 16 (FR)
(72) Inventor: Pardo, Raul, 75014 Paris (FR); Humbert, Sandrine, 91400 Orsay (FR); Saudou, Frédéric, 91400 Orsay (FR)
(74) Representative: Gallois, Valérie

(57) **Abstract**

The present invention relates to a method for treating patients having Huntington's disease by a drug increasing the phosphorylation of huntingtin at position S421, thereby decreasing the polyQ-huntingtin-induced toxicity.
Useful compounds are selected from the group consisting of cyclosporin A, FK506, FK520, L685, 818, FK523, 15-0-DeMe- FK-520, Lie120, fenvalerate, resmethrin, cypermethrin, deltamethrin. A method for selecting, identifying or screening a compound useful for treating a subject having Huntington's disease is also disclosed.

## Description

### FIELD OF THE INVENTION

The present invention relates to methods for treating Huntington's disease, pharmaceutical compositions useful in such methods, and screening methods for identifying a compound useful for treating Huntington's disease.

### BACKGROUND OF THE INVENTION

Huntington's disease (HD) results from genetically programmed degeneration of neurons, in certain areas of the brain. The prevalence of the of HD in occidental world is 1 out of 10 000 people. This degeneration causes uncontrolled movements, loss of intellectual faculties, and emotional disturbance. HD is an autosomal dominant neurodegenerative disease. A person who inherits the HD gene will sooner or later develop the disease. Some early symptoms of HD are mood swings, depression, irritability or trouble driving, learning new things, remembering a fact, or making a decision. As the disease progresses, concentration on intellectual tasks becomes increasingly difficult and the patient may have difficulty feeding himself or herself and swallowing. The rate of disease progression and the age of onset vary from person to person. A genetic test, coupled with a complete medical history and neurological and laboratory tests, help physician's diagnose HD. Presymptomic testing is available for individuals who are at risk for carrying the HD gene. In 1 to 3 percent of individuals with HD, no family history of HD can be found.

At this time, there is no way to stop or reverse the course of HD. Drugs used to treat HD only alleviate symptoms and have side effects such as fatigue, restlessness, or hyperexcitability. Therefore, there is a strong need for HD treatment.

HD is caused by an abnormally expanded CAG tract in the coding region of the *IT15* gene located in chromosome 4p16.3, which encodes a polyglutamine tract in the protein huntingtin (htt). Huntingtin becomes toxic when it contains an abnormal polyQ expansion. PolyQ-huntingtin is cleaved in the cytoplasm, and thereafter translocates to the nucleus where it forms ubiquitin immunopositive nuclear aggregates. When in the nucleus, polyQ-huntingtin induces transcriptional dysregulation and neuronal death through a gain of function mechanism (Ross, 2002;). A loss of the protective functions of huntingtin could act concomitantly and/or synergistically with the gain of new toxic functions (Cattaneo et al., 2001). In agreement, dysregulation of BDNF transcription is linked to the loss of huntingtin normal function (). In addition, when huntingtin contains the polyQ expansion, its ability to transport BDNF-containing vesicles and to promote neuronal survival is lost (Gauthier et al., 2004). Finally, aggregates are also found in neurites and could also participate in neuronal dysfunction by altering the microtubules network dynamics and/or axonal transport Charrin et al., 2005).

Several post-translational modifications such as proteolysis, ubiquitination and sumoylation modify the toxicity of huntingtin (. Another post-translational modification that plays an important role in disease is phosphorylation. In particular, the Ser/Thr kinases, Akt and the *Serum and Glucocorticoid-induced Kinase,* SGK, phosphorylate huntingtin at serine 421 (S421) (Humbert et al., 2002; Rangone et al., 2004; Warby et al., 2005). Phosphorylation at S421 subsequently abolishes polyQ-huntingtin induced toxicity in a cellular model of HD (Humbert et al., 2002; Rangone et al., 2004). However, it is not clear whether phosphorylation of S421 is crucial *in vivo.* In addition, huntingtin is phosphorylated at serine 434 by the cyclin-dependent kinase Cdk5 and this reduces its cleavage by caspases (Luo et al., 2005).

Immunophillin ligands, such as FK506 and cyclosporin, have been recently described as a putative therapeutic strategy for neuroregeneration and neuroprotection (Klettner and Herdegen, 2003; Pong and Zaleska, 2003). Therefore, immunophillin ligands have been suggested for treating neurodegenerative diseases. The immunophillin ligands have two kind of activity : 1) they inhibit peptidyl-prolyl isomerase (PPI or rotamase); 2) they inhibit calcineurin, thereby having an immunosuppressive function. However, the role of these two kind of activity in neuroprotection and neuroregeneration is still not clear.

Calcineurin is involved in the activation of neuronal nitric oxide synthase (nNOS). nNOS produces nitric oxide (NO) which leads to structural damage to lipids, proteins, and DNA. Otherwise, calcineurin dephosphorylates BAD and enhances its hetero-dimerization with Bcl-X_{L}, leading to apoptosis. Therefore, inhibition of calcineurin can block the downstream mediators of cell death. Similarly, stabilization of mitochondrial function can also mediate neuroprotection.

Non-immunosuppressant immunophilin ligands (e.g., GPI-1046 and V10,367), devoid of calcineurin inhibition activity, have been developed for treating neurological disorders. In particular, clinical trials with GPI-1046 and V10,367 for treating Parkinson's disease have failed.

No specific data for HD treatment has been reported except Almeida et al (2004). This article reports an *in vitro* study on cortical cells treated by 3NP (3-nitropropionic acid) (a chemical model of HD which does not reproduce huntingtin mutation). 3NP treatment induces cell death and this cell death can be blocked by FK506 through an unclear mechanism not directly linked to the phosphoryaltion of huntingtin at positions S421.

### SUMMARY OF THE INVENTION

The inventors demonstrate that phosphorylation of huntingtin at position S421 is neuroprotective *in vivo* and that calcineurin (CaN) dephosphorylates S421. Inhibition of CaN activity leads to an increased phosphorylation of huntingtin at S421 and prevents polyQ-huntingtin-induced death of neurons. Consequently, the inventors demonstrate the interest of a drug increasing the phosphorylation of huntingtin at position S421 as a therapeutic approach to treat HD by decreasing the polyQ-huntingtin-induced toxicity.

Therefore, the present invention concerns the use of a drug increasing the phosphorylation of huntingtin at position S421 for the manufacture of a medicament for blocking or reducing the toxicity of polyQ-huntingtin in a subject having Huntington's disease. In particular, the drug is aiming to block or reduce the neuronal death induced by polyQ-huntingtin. The drug increasing the phosphorylation of huntingtin at position S421 can be a drug increasing the activity of the kinase Akt, protein kinase A, Polo kinase 1, AuroraA and AuroraB and/or SGK. In a preferred embodiment, the drug increasing the phosphorylation of huntingtin at position S421 is a drug inhibiting the dephosphorylation of huntingtin at position S421. More preferably, the drug inhibiting the dephosphorylation of huntingtin at position S421 is a calcineurin inhibitor or a drug inhibiting the interaction between calcineurin and huntingtin or a dominant interfering form of calcineurin. In the most preferred embodiment, the drug inhibiting the dephosphorylation of huntingtin at position S421 is a calcineurin inhibitor. In one embodiment, the calcineurin inhibitor is selected from the group consisting of cyclosporin A, FK506, FK520, L685,818, FK523, 15-0-DeMe-FK-520, Lie120, fenvalerate, resmethrin, cypermethrin, deltamethrin and an analogue thereof. Preferably, the calcineurin inhibitor is selected from the group consisting of FK506, cypermethrin, deltamethrin and an analogue thereof. More preferably, the calcineurin inhibitor is FK506. Alternatively, the calcineurin inhibitor can be a RNA interference specific of calcineurin. The subject having Huntington's disease carries the Huntington's disease mutation. In particular, the HD mutation is an abnormal expansion (superior to 35) of the CAG repeat in the coding region of the IT15 gene.This subject can be presymptomatic or can also have developed the symptoms of HD. In this case, the drug increasing the phosphorylation of huntingtin at position S421 can be used in combination with a drug alleviating symptoms of Huntington's disease.

In a further embodiment, the present invention concerns a method for selecting, identifying or screening a compound useful for treating a subject having Huntington's diseases, comprising the selection or identification of a compound capable of increasing the phosphorylation of huntingtin at position S421.

### LEGEND TO THE FIGURES

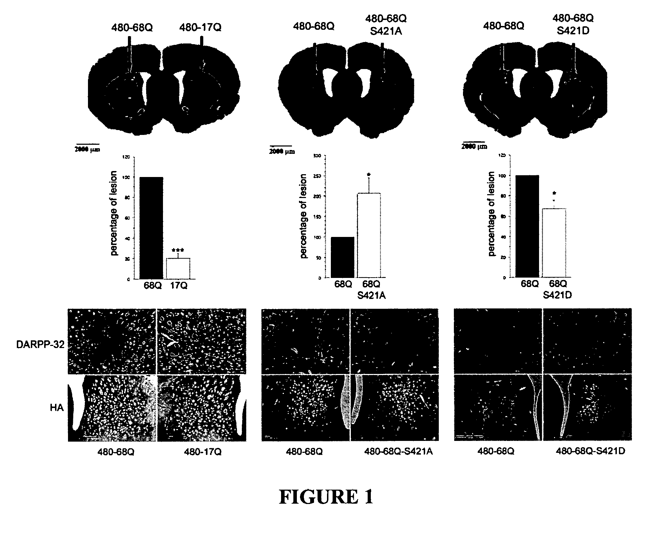

**Figure 1.** Phosphorylation of polyQ-huntingtin at S421 is neuroprotective *in vivo.* Rat striata were injected with 480-17Q (wild-type), 480-68Q (polyQ), 480-68Q-S421A or 480-68Q-S421D lentiviruses. The polyQ-huntingtin-induced lesions in rat striatum were analyzed 24 weeks after injection. Immunohistochemical analysis with DARPP-32 antibody were performed and the size of the lesions were determined for all constructs and expressed as a percentage of the lesion induced by the 480-68Q construct. As expected, a drastic loss of DARPP-32-immunoreactive neurons was observed in the 480-68Q-infected striatum whereas expression of the wild-type protein had no effect (Paired Student t-test; t_{[7]} = 6.95 ; ***P < 0.001). Absence of phosphorylation at S421 (480-68Q-S421A) resulted in an increased size of the DARPP-32-immunodepleted region (Paired Student t-test; t_{[6]} = 3.17 ; *P < 0.05). In contrast, constitutive phosphorylation at S421 (480-68Q-S421D) significantly reduced the size of the DARPP-32-immunodepleted region (Paired Student t-test; t_{[7]} = 3.46 ; *P < 0.05). Anti-HA immunohistochemical analysis revealed that all constructs were expressed at similar levels (lower panels).

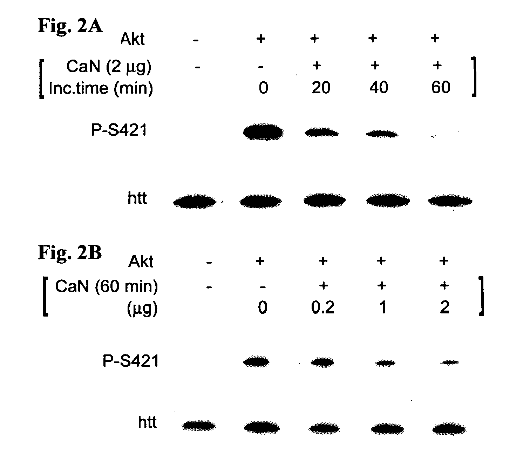

**Figure 2.** Calcineurin dephosphorylates S421 *in vitro.* **Fig. 2A,** Time-course and **Fig. 2B,** dose-dependent dephosphorylation of S421 by purified calcineurin. A GST-coupled fragment of human huntingtin (aminoacids 384-467) was phosphorylated with recombinant Akt (0.2 µg/sample) and then incubated with 2 µg (A) or different quantities (B) of purified calcineurin for the indicated times (A) or 60 min (B). Immunoblotting experiments were performed using anti-phospho-huntingtin-S421-763 or anti-huntingtin (1259) antibodies.

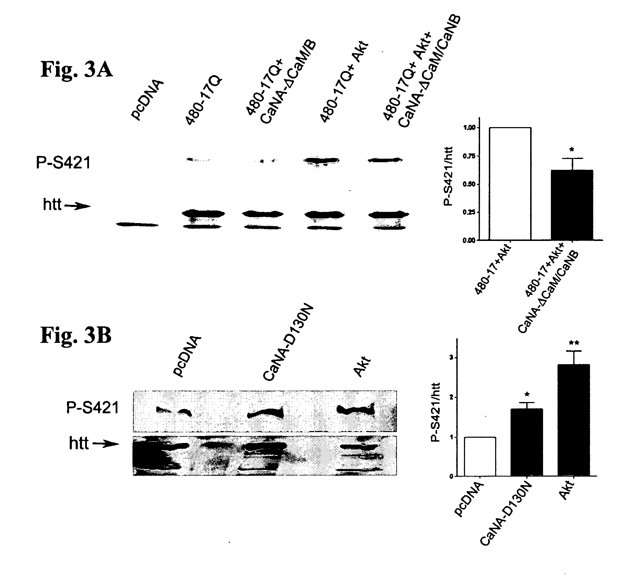

**Figure 3.** Calcineurin dephosphorylates S421 of huntingtin in cells. **Fig. 3A,** Mouse striatal cells (+/+ cells) were transfected with a N-terminal fragment of huntingtin (480-17Q), Akt a constitutive active form of calcineurin (CaNA-ΔCaM /CaNB) or the corresponding empty vector, and processed for Western blot analysis with anti-phospho-huntingtin-S421-714 or anti-huntingtin (1259) antibodies. Data from three independent experiments revealed that constitutive active form of calcineurin significantly reduced the phosphorylation of S421 triggered by Akt (Student's t test; t_{[3]} = 3.63; *P < 0.05). **Fig. 3B,** SHSY-5Y cells were transfected with a catalytic-dead form of calcineurin (CaNA-D130N), Akt or the corresponding empty vector and analyzed with anti-phospho-huntingtin-S421-763. Data are from three independent experiments (Analysis of variance (ANOVA); F_{[2,6]} = 17.31; P = 0.032). The phosphorylation of endogenous huntingtin on S421 was significantly increased by the catalytic-dead construct of calcineurin (Student's t test; t_{[4]} = 4.34; *P < 0.05) and by Akt (t_{[4]} = 5. 26; **P < 0.01).

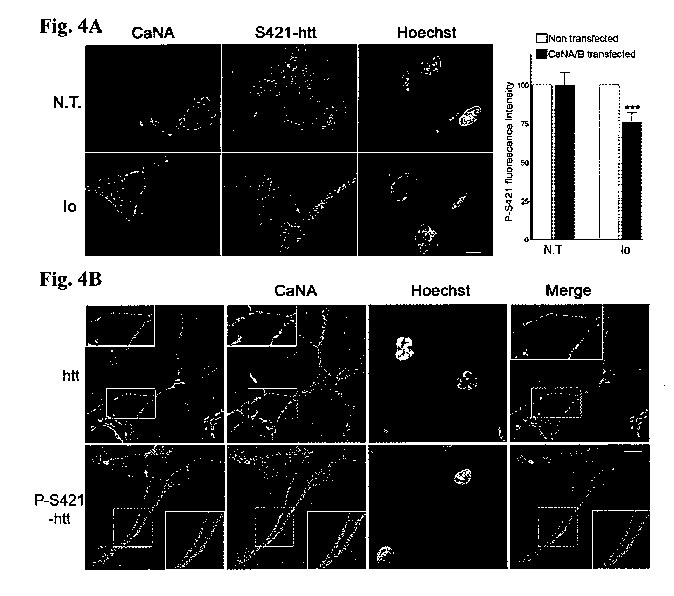

**Figure 4.** Huntingtin colocalizes with calcineurin and is dephosphorylated upon Ca²⁺-induced calcineurin activation. **Fig. 4A,** Ionomycin (Io, lower panels) decreased huntingtin S421 phosphorylation in cells transfected with wild-type CaNA/CaNB. SHSY-5Y cells were cotransfected with wild-type forms of CaNA/CaNB, in comparison to non treated cells (N.T., top panels). The graph corresponds to the quantification of the mean fluorescence intensity of the phospho-S421-htt (763 antibody) signal issued from 32 cells (ANOVA; F_{[3,28]}=7,05 ; P = 0.0011) and shows that ionomycin-induced dephosphorylation of huntingtin was significant (Fishers's post hoc test; ***P < 0.001). **Fig. 4B,** Huntingtin colocalized with the catalytic subunit of calcineurin (CaNA) to vesicular structures. Immunostaining for huntingtin (1259 antibody), phospho-S421-huntingtin (714 antibody) and CaNA in striatal neurons. A substantial colocalization was observed in vesicular structures along neurites (see enlargements). Scale bars, 5µm.

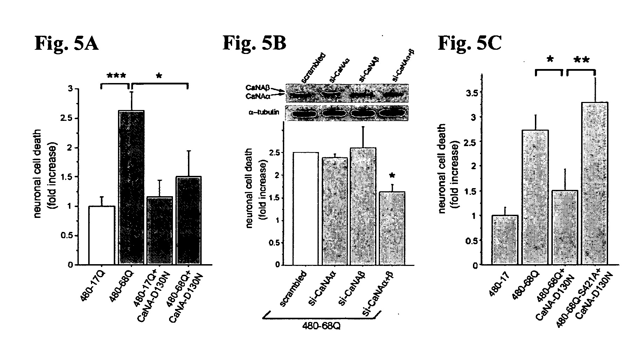

**Figure 5.** A dominant-interfering form of calcineurin inhibits polyQ-huntingtin-induced toxicity in striatal neurons in a S421 phosphorylation-dependent manner. **Fig. 5A,** Wild-type huntingtin (480-17Q) or polyQ-huntingtin (480-68Q) were cotransfected with expression vectors encoding a catalytic-dead form of calcineurin (CaNA-D130N) or the corresponding empty vector in striatal neurons. Data from four independent experiments (ANOVA; F_{[3,56]} = 6.99; P = 0.0004) revealed that CaNA-D130N significantly reduced polyQ-huntingtin induced cell death (Fishers's post hoc test; *P < 0.05, ***P < 0.0001). **Fig. 5B,** Striatal neurons were nucleofected with siRNA directed against CaNAα, CaNAβ or both isoforms and 30 hours later transfected with 480-68Q as in Fig. 5A. Combination of siRNA directed against both isoforms of CaN efficiently reduced CaNA expression as detected by Western blot analysis. Data from three independent experiments (ANOVA; F_{[3,6]}=5.33; P = 0.039) demonstrated a protective effect towards polyQ-dependent toxicity (Fishers's post hoc test; *P < 0.05). Fig. 5C, Striatal neurons were cotransfected with 480-68Q or a non-phosphorylable form (480-68Q-S421A) and CaNA-D130N. Data from four independent experiments (ANOVA; F_{[2,41]}=4.30; P = 0.0004) demonstrated that the catalytic-dead form of calcineurin did not prevent from polyQ-dependent cell death triggered by the 480-68Q-S421A construct (Fishers's post hoc test; *P < 0.05, **P < 0.01) indicating that neuroprotection was dependent on the phosphorylation of S421.

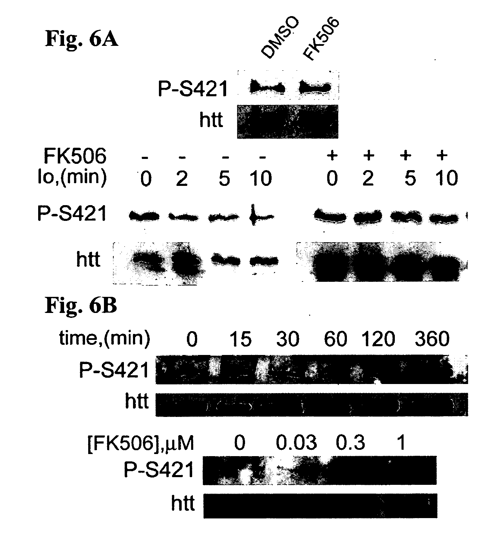

**Figure 6.** Inhibition of calcineurin by FK506 prevents the dephosphorylation of S421. **Fig. 6A,** Treatment with FK506 (20 min, 1 µM) in Jurkat cells increased the phosphorylation of endogenous huntingtin at S421 (left). The calcium ionophore ionomycin (Io, 2.5 µM) induced a rapid dephosphorylation of S421 in Jurkat cells. Io-elicited S421 dephosphorylation was prevented by FK506 (1 µM) (right). Whole-cell extracts of treated Jurkat T cells were analyzed with the anti-phospho-huntingtin-S421-763 and the anti-huntingtin (1259) antibodies. **Fig. 6B,** Time-course and dose-dependent phosphorylation of S421 following FK506 treatment of rat striatal neurons. Neurons were treated with 1 µM FK506 for different times (top) or with the indicated concentrations for 2 h (bottom). Whole-cell extracts were analyzed with the mouse phospho-specific (anti-phospho-huntingtin-S421-714) and the anti-huntingtin (1259) antibodies.

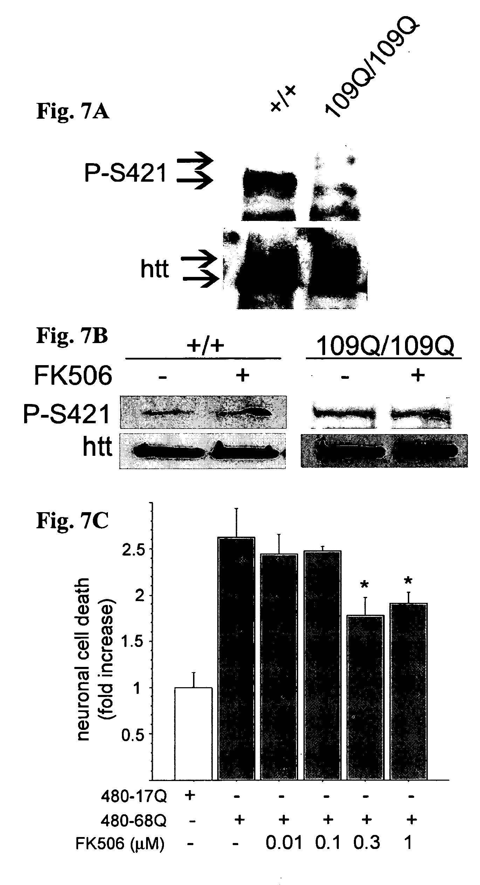

**Figure 7.** 109Q/109Q cells show reduced phosphorylation of huntingtin on S421 that can be increased by FK506. **Fig. 7A,** Whole-cell extracts of immortalized knock-in cells issued from wild-type (+/+) or mutant mice (109Q/109Q) were analyzed by western-blot with the mouse phospho-specific and anti-huntingtin (1259) antibodies. **Fig. 7B,** FK506 (0.2 µM, 48 hr) increased the phosphorylation of S421 on wild-type and polyQ-huntingtin. **Fig. 7C,** FK506 protects neurons from polyQ-mediated cell death. Striatal neurons were transfected with 480-68Q in the presence of different doses of FK506 or vehicle. Data from three independent experiments (ANOVA; F_{[5,68]} = 8.02; P < 0.0001) revealed that FK506 significantly reduced neuronal death induced by 480-68Q (Fishers's post hoc test; *P < 0.05).

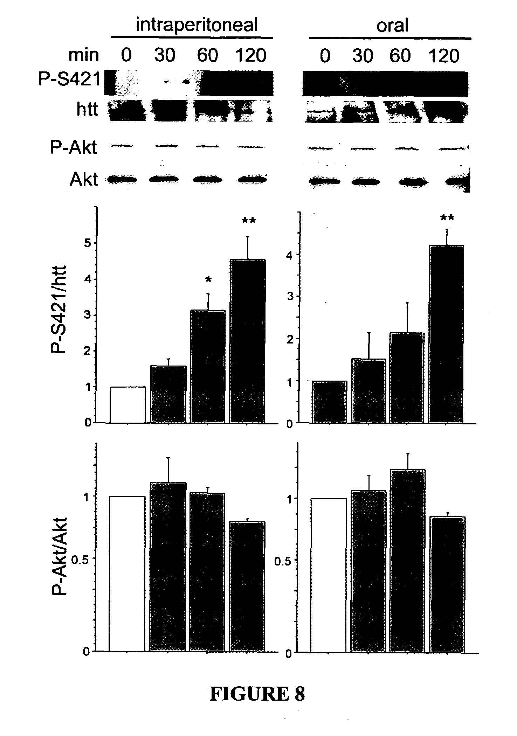

**Figure 8.** Administration of FK506 to mice results in increased phosphorylation of S421 in the brain. FK506 was administered intraperitoneally or orally to mice (5mg/kg). Animals were sacrificed at the indicated times post-administration, brains were dissected, homogenized and processed for Western blot analysis. Whole brain extracts were probed with anti-phopho-S421, anti-phospho-S473-Akt, anti-total huntingtin and anti-total Akt antibodies. Bottom panels correspond to the densitometic analysis of two independent experiments performed in duplicate. A total of 18 mice were sacrified. From top to bottom and from left to right: (ANOVA; F_{[3,4]} = 11.52, P = 0.019); (ANOVA; F_{[3,4]} = 8.694, P = 0.031); (ANOVA; F_{[3,4]} = 1,668, P = 0.31); (ANOVA; F_{[3,4]} = 0.97, P = 0.4); *P < 0.05, **P < 0.01.

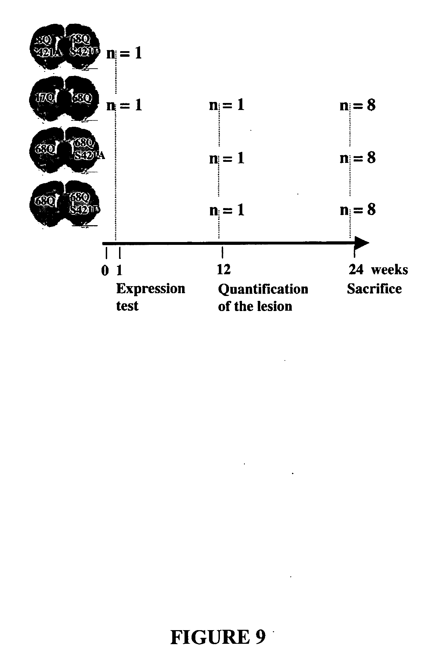

**Figure 9.** Diagram summarizing the injection protocol of the various 480 fragments. The experience was performed on 29 animals. One week after injection, 2 rats were analyzed to control the expression of huntingtin. 12 weeks after injection, the lesion was evaluated in one animal of each group. As the lesion was not significant at this time point, the final evaluation of all the animals was performed at 24 weeks after injection. The number of glutamines (17Q or 68Q) and the mutation of the serine 421 for each injected fragment of huntingtin are indicated on each hemisphere on the rat brain sections. n indicates the number of animals per condition.

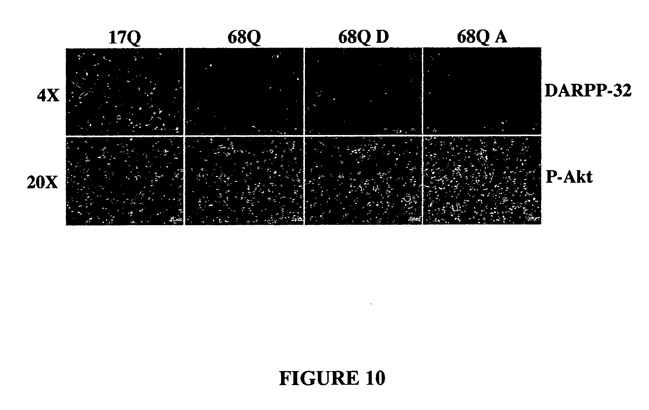

**Figure 10.** Rat striata injected with 480-17Q (wild-type), 480-68Q (polyQ), 480-68Q-S421A or 480-68Q-S421D lentiviruses were analyzed 24 weeks after injection by immunohistochemistry using anti-DARPP-32 and anti-phospho-S473-Akt (9277, Cell Signaling Technology) antibodies. Although a down-regulation of DARPP-32 is observed in the polyQ-huntingtin infected region, no signs of neuronal loss were observed as indicated by the presence of neurons immunopositive for the phosphorylated form of Akt.

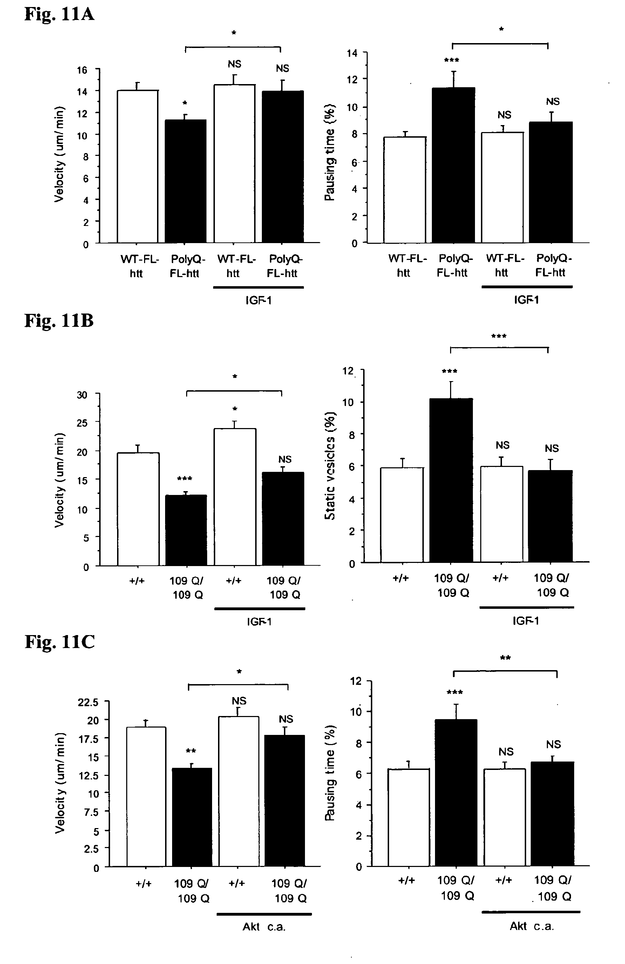

**Figure 11.** IGF-1 and Akt rescues polyQ-huntingtin-induced alteration of transport in primary neurons and in HD neuronal cells. **Fig. 11A,** IGF-1 significantly increases the mean velocity of BDNF-containing vesicles (left) and decreases their pausing time (right) in primary cultures of cortical neurons that express FL-htt-75Q. **Fig. 11B,** The altered BDNF transport in 109Q/109Q cells is ameliorated by IGF-1 that increases velocity (left) and decreases pausing time (right). **Fig. 11C,** Akt completely inhibits transport deficiency in 109Q/109Q cells by significantly increasing velocity (left) and decreasing pausing time (right) (*P<0.05).

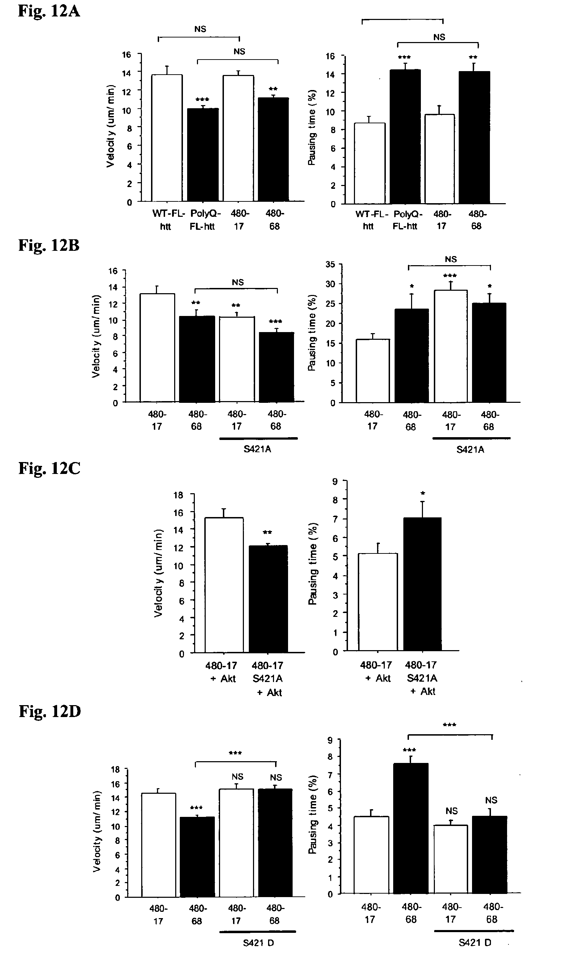

**Figure 12.** Phosphorylation of huntingtin at S421 regulates transport and restores polyQ-huntingtin loss of function. **Fig. 12A,** Measurements of mean velocity (left) and pausing time (right) of BDNF-containing vesicles in control condition (BDNF alone), and after transfection of FL-htt with 17 or 75Q, or the N-terminal fragment 480 with 17 or 68Q in neuronal cells demonstrate that a N-terminal fragment of htt of 480 amino acid is able to stimulate transport but is altered when containing the polyQ expansion. **Fig. 12B,** The capacity of the wild-type 480 fragment (480-17Q) to promote transport is lost when S421 is not phosphorylated as demonstrated by the significant decrease in velocity (left) and increase in the pausing time (right) of BDNF-containing vesicles in the presence of the 480-S421A construct. **Fig. 12C,** The capacity of Akt to enhance BDNF transport is mediated by the phosphorylation of S421 of htt as the mean velocity of BDNF vesicles in presence of Akt is decreased and the pausing time of vesicles is increased with the 480-17Q-S421A construct in comparison to the 480-17Q construct. Fig. 12D. S421D mutation in 480-68Q construct completely rescues mean velocity of vesicles (left) and pausing time (right) in comparison to the 480-68Q construct. (*P<0.05).

### DETAILED DESCRIPTION OF THE INVENTION

Using a rat model of HD based on lentiviral-mediated expression of a polyQ-huntingtin fragment in the striatum, the inventors demonstrate that phosphorylation of S421 is neuroprotective *in vivo.* They also demonstrate that calcineurin (CaN), a calcium/calmodulin-regulated Ser/Thr protein phosphatase, dephosphorylates S421 *in vitro* and in cells. Importantly, inhibition of CaN activity either by overexpression of a dominant-interfering form, by RNA interference or by the specific inhibitor FK506, leads to an increased phosphorylation of huntingtin at S421 and prevents polyQ-mediated death of striatal neurons. Finally, the inventors show that administration of FK506 to mice increases huntingtin S421 phosphorylation in brain. Collectively, these data highlight the importance of CaN in the modulation of S421 phosphorylation and suggest the potential use of CaN inhibition as a therapeutic approach to treat HD as it decreases the polyQ-induced toxicity.

Calcineurin (CaN), also known as protein phosphatase 2B (PP2B), is a phosphoprotein Ser/Thr phosphatase activated physiologically by Ca2+-calmodulin (for a review see Mansuy, 2003). Therefore, it couples intracellular calcium to the dephosphorylation of selected substrates, which include transcription factors *(Nuclear Factor of Activated T cells,* NFAT), ion channels (inositol-1,4,5 triphosphate receptor), proteins involved in vesicular trafficking (amphyphysin, dynamin), scaffold proteins (AKAP79) and phosphatase inhibitors (DARPP-32, inhibitor-1). CaN is present in all tissues in mammals, with notably high levels in brain, some studies indicating that it may account for 1% of the total protein content of the brain. The catalytic subunit is mainly expressed in the cortex, striatum and the hippocampus. Within the central nervous system, CaN activity has been involved in synaptic plasticity, as it is considered a negative constraint for long-term memory. In nerve terminals, CaN triggers synaptic vesicle endocytosis by dephosphorylating vesicular and plasma membrane proteins in response to Ca2+. Finally, CaN is inhibited by drugs such as cyclosporin A and FK506 through the binding of these drugs to their appropriate receptors (immunophilins); Hamawy, 2003). FK506 and its derived immunophilin ligands, unlike cyclosporin, can readily cross the blood-brain barrier (Pong and Zaleska, 2003).

The inventors have previously shown that huntingtin is a processivity factor for intracellular transport of vesicles such as *Brain Derived Neurotrophic factor* (BDNF). In HD, the transport of BDNF vesicles in cortical neurons is altered leading to a reduction of trophic support and neuronal toxicity in the striatum. In the present invention, the inventors demonstrate that huntingtin phosphorylation at S421 by the IGF-1/Akt pathway rescues transport altered by the polyQ expansion and, by promoting an outward BDNF flow increases neurotrophic support. These results elucidate the neuroprotective effect of S421 phosphorylation in HD and suggest that drugs enhancing huntingtin phosphorylation are of therapeutic interest since phosphorylation of S421 rescues huntingtin function in transport. In addition, the inventors demonstrate that huntingtin phosphorylation by Akt at serine 421 regulates the ability of polyQ-huntingtin but also normal huntingtin to promote transport.

The present invention concerns the use of a drug increasing the phosphorylation of huntingtin at position S421 for the manufacture of a medicament for blocking or reducing the toxicity of polyQ-huntingtin in a subject having Huntington's disease. Preferably, the drug is aiming to block or reduce the neuronal death induced by polyQ-huntingtin. The drug is also aiming to restore the huntingtin's activity in the axonal transport.

The present invention also concerns a method for treating a subject having Huntington's disease by administering a therapeutic amount of a drug increasing the phosphorylation of huntingtin at position S421, thereby blocking or reducing the toxicity of polyQ-huntingtin. In particular, the drug is aiming to block or reduce the neuronal death induced by polyQ-huntingtin. The drug is also aiming to restore the huntingtin's activity in the axonal transport.

The subject having Huntington's disease is a subject that carries the HD mutation carrying a Huntington disease gene. The mutation that causes Huntington's disease is an anormal CAG repeat expansion in the huntingtin gene (also called *IT15* gene). Indeed, in normal individuals, the repeat occurs between 6 and 35 times. In a subject having HD, the repeat occurs more than 36 times, generally from 40 times to more than 80 times. This mutation results in a polyglutamine (poly-Q) expansion in the protein named huntingtin.

The subject having Huntington's disease can be presymptomatic. The presymptomatic subject is identified by a genetic test. US patent 4,666,828 discloses a method for detecting the presence in a subject of the gene for Huntington's disease comprising the analysis of a DNA polymorphism linked to Huntington's Disease on the human chromosome 4, in particular a RFLP. In a preferred embodiment, the test analyzes DNA directly for the presence of the Huntington's disease mutation (Kremer et al, 1994). Of course, any test allowing to determine if a subject has an anormal huntingtin protein or gene is contemplated in the present invention.

Accordingly, the present invention concerns a method for treating HD subject comprising: detecting the presence in the subject of the HD mutation; and, administering a therapeutic amount of a drug increasing the phosphorylation of huntingtin at position S421 to the subject having the HD mutation, thereby blocking or reducing the neuronal death induced by polyQ-huntingtin. In particular, detecting the presence in the subject of the HD mutation comprises the determination of the number of CAG repeat in the IT15 gene. Subject having more than 36 repeats, preferably more than 40 repeats, is considered as having the HD mutation. Alternatively, detecting the presence in the subject of the HD mutation can also be done at the huntingtin protein level by determining the size of the polyglutamine expansion.

Alternatively, the subject having Huntington's disease can have HD symptoms. HD symptoms are well-kown by the one skilled in the art. For instance, they comprise uncontrolled movements, loss of intellectual faculties (e.g., dementia), and emotional disturbance (e.g., depression). In this case, the drug increasing the phosphorylation of huntingtin at position S421 can be used in combination with a drug alleviating symptoms of Huntington's disease. For example, such drugs treating HD symptoms can be antipsychotics (haloperidol, chlorpromazine, olanzapine), antidepressants (fluoxetine, sertraline hydrochloride, nortriptyline), tranquilizers (benzodiazepines, paroxetine, venlafaxin, beta-blockers), mood-stabilizers (lithium, valproate, carbamazepine), and Botulinum toxin.

Preferably, the subject is a primate, more preferably a higher primate, most preferably a human.

The treatment of a subject having HD can result in an improved motor coordination, an increased survival, the prevention of Huntington's disease from occurring in a presymptomatic HD subject, the retardation or arrest of HD development and/or a regression of HD. Indeed, the treatment according to the present invention results in blocking or reducing the toxicity of polyQ-huntingtin, thereby blocking or reducing the neuronal death induced by polyQ-huntingtin. Therefore, at an early stage of HD such as presymptomatic stage, the HD development should be prevented. At a later stage of HD, the HD development should be inhibiting or slowing down. The inventors also showed in the present invention that the increased phosphorylation of huntingtin at S421 results in the full or partial recovery of its activity in the axonal transport, in particular BDNF transport which is linked to the anti-apoptotic action of normal huntingtin.

Due to the increased anti-neuronal death role of huntingtin following the treatment according to the present invention through its increased axonal transport capacity, the present invention further contemplates the use of a drug increasing the phosphorylation of huntingtin at position S421 for the manufacture of a medicament for increasing the axonal transport by huntingtin in a subject suffering from or at risk of a neurodegenerative disease. The neurodegenerative disease can be selected from the group consisting of Alzheimer's disease, amyotrophic lateral sclerosis, and Parkinson's disease. The present invention also contemplates a method for treating a subject suffering from or at risk of a neurodegenerative disease comprising administering a therapeutic amount of a drug increasing the phosphorylation of huntingtin at position S421, thereby increasing the axonal transport by huntingtin. The increased axonal transport by huntingtin is aiming to have an anti-apoptotic effect.

A drug increasing the phosphorylation of huntingtin at position S421 can be a drug increasing the activity of the kinase Akt, protein kinase A, Polo kinase 1, AuroraA, AuroraB and/or SGK. However, in a preferred embodiment, the drug increasing the phosphorylation of huntingtin at position S421 is a drug inhibiting the dephosphorylation of huntingtin at position S421. More preferably, the drug inhibiting the dephosphorylation of huntingtin at position S421 is a calcineurin inhibitor or a drug inhibiting the interaction between calcineurin and huntingtin. In the most preferred embodiment, the drug inhibiting the dephosphorylation of huntingtin at position S421 is a calcineurin inhibitor.

Calcineurin inhibitors include, but are not limited thereto, cyclosporin A (Novartis International AG, Switzerland), FK506 (Fujisawa Healthcare, Inc., Deerfield, IL, USA), FK520 (Merck & Co, Rathway, NJ, USA), L685,818 (Merck & Co), FK523, 15-0-DeMe-FK-520 (Liu, Biochemistry, 31:3896-3902 (1992)), Lie120, fenvalerate (Merck & Co), resmethrin (Merck & Co), cypermethrin (Merck & Co) and deltamethrin (Merck & Co). WO2005087798 describes cyclosporine derivative inhibiting calcineurin.

Calcineurin is a heterodimer composed of a catalytic subunit (Calcineurin A) and a regulator subunit (Calcineurin B). Then, the activity of calcineurin can also be inhibited by blocking its expression, in particular the expression of one of its subunit. In a preferred embodiment, the activity of calcineurin can also be inhibited by blocking the expression of the regulator subunit B. In an alternative preferred embodiment, the activity of calcineurin can also be inhibited by blocking the expression of the regulator subunit A, either CaNAα or CaNAβ. The expression can be blocked by any mean known by one skilled in the art, e.g., by chemically synthesized oligonucleotides such as antisense oligonucleotides, ribozymes, short interfering RNA (siRNA) and short hairpin RNA (shRNA). Antisense oligonucleotides are short single-strand molecules that are complementary to the target mRNA and typically have 10-50 mers in length, preferably 15-30 mers in length, more preferably 18-20 mers in length. Antisense oligonucleotides are preferably designed to target the initiator codons, the transcriptional start site of the targeted gene or the intron-exon junctions (for review, Kurreck, 2003). Ribozymes are single stranded RNA molecules retaining catalytic activities. The mechanism of ribozyme action involves sequence specific interaction of the ribozyme molecule to complementary target RNA, followed by an endonucleolytic cleavage. The ribozyme is engineered to interact with the target RNA of interest comprising a cleavage NUH triplet, preferentially GUC (for review, Usman et al., 2000). siRNA are usually 21 or 23 nucleotides long, with a 19 or 21 nucleotides duplex sequence and 2 nucleotides-long 3' overhangs. shRNA are designed with the same rules than for a sequence encoding a siRNA excepting several additional nucleotides forming a loop between the two strands of the siRNA. (For review, see Mittal, 2004). In a particular embodiment, the inhibiting oligonucletide is expressed by a vector, preferably a viral vector comprising a contruct allowing the expression of inhibiting oligonucleotides. For instance, the viral vector can be an adenovirus, an AAV, a lentivirus or a HSV. Examples disclosed in detailed one way to performed the calcineurin inhibition by siRNA.

In a particular embodiment, the present invention concerns a RNA interference specific of calcineurin as a medicament. In a preferred embodiment, the present invention concerns the use of a RNA interference specific of calcineurin for the manufacture of a medicament for treating Huntington's disease. Such a RNA interference is directed against CaNAα (NM_000944) and/or CaNAβ (NM_021132).

Calcineurin inhibitor can also be a dominant-interfering form of calcineurin, in particular of CaNA and/or of CaNB. The dominant-interfering form of calcineurin can be for example CaNA-D130N. Therefore, the dominant-interfering form of calcineurin is expressed by a vector, preferably a viral vector comprising a contruct allowing its expression. For instance, the viral vector can be an adenovirus, an AAV, a lentivirus or a HSV.

Alternatively, the activity of calcineurin can be inhibited by a drug that inhibits the interaction between the calcineurin subunits, in particular the interaction between subunits A and B. The activity of calcineurin can be inhibited by a drug that inhibits the interaction between the calcineurin and calmodulin. Calcineurin inhibition can also be obtained by activation of endogenous inhibitors of calcineurin, including cabin1, calcipressins and AKAP79.

By "analogue" of a drug is intended a compound having similar structural features and having the same biological activity, in particular which inhibits calcineurin.

Other drugs inhibiting the calcineurin can be identified by screening methods already disclosed in the art. As illustration, the US patents 6,875,581 and 6,338,946 describe screening methods useful for identifying modulators of calcineurin activity.

Therefore, the present invention also concerns methods for selecting, identifying or screening a compound useful for treating a subject having Huntington's diseases, comprising the selection or identification of a compound capable of increasing the phosphorylation ofhuntingtin at position S421.

The method can comprise :
a) providing a huntingtin protein or a fragment thereof of at least 50 consecutive amino acids comprising S421, said huntingtin protein or fragment thereof having a phosphorylated S421;
b) providing a calcineurin;
c) contacting a candidate compound with said huntingtin protein or fragment thereof and said calcineurin; and,
d) selecting the candidate compound that inhibits the dephosphorylation of huntingtin S421 by calcineurin.

Alternatively, the method can comprise :
a) contacting a candidate compound with a cell expressing a huntingtin protein and comprising a kinase which phosphorylates huntingtin at position S421 and a calcineurin;
b) assessing the amount of huntingtin phosphorylated at position S421 and/or the amount of huntingtin which is not phosphorylated at position S421; and,
c) selecting the candidate compound that increases the phosphorylation of huntingtin at position S421 in comparison with a control cell which has not been contacted with the candidate compound.

In a preferred embodiment, the phosphorylation of huntingtin at position S421 is detected with a antibody specific huntingtin phosphorylated at position S421, in particular by Western Blot. Preferably, the cell is a neuron, in particular a stratial neuron. However, any kind of cell is contemplated in the present invention.

The drug inhibiting the calcineurin may be of various origin, nature and composition. It may be any organic or inorganic substance, such as a lipid, peptide, polypeptide, nucleic acid, small molecule, etc., in isolated or in mixture with other substances. In a preferred embodiment, the drug is a small molecule. In an other preferred embodiment, the drug is a peptide or a polypeptide. In an additional embodiment, the drug is a nucleic acid, e.g., an antisense, a siRNA, a ribozyme. In another embodiment, the drug is an aptamer. In a preferred embodiment, the drug inhibiting calcineurin crosses the blood-brain barrier.

The drug used in the present invention can be formulated as a pharmaceutical composition generally comprising a pharmaceutically acceptable carrier. By a pharmaceutically acceptable carrier is intended a carrier that is physiologically acceptable to the treated mammal while retaining the therapeutic properties of the drug with which it is administered. For example, a pharmaceutically acceptable carrier can be physiological saline solution. Other pharmaceutically acceptable carriers are known to one skilled in the art and described for instance in Remington: The Science and Practice of Pharmacy (20th ed., ed. A.R. Gennaro AR., 2000, Lippincott Williams & Wilkins).

The compositions of the present invention may be administered orally, parenterally, by inhalation spray, topically, rectally, nasally, buccally, vaginally or via an implanted reservoir. The term "parenteral" as used herein includes subcutaneous, intravenous, intramuscular, intra-articular, intra-synovial, intrasternal, intrathecal, intrahepatic, intralesional and intracranial injection or infusion techniques. Preferably, the compositions are administered orally, intraperitoneally or intravenously. For drugs that do not pass the blood-brain barrier, the drugs need to be injected into the brain, for example by a stereotaxic injection.

By a "therapeutic amount" is intended an amount of a drug that is sufficient to increase the phosphorylation of huntingtin at position S421. The effective amount of a drug varies depending upon the administration mode, the age, body weight, sex and general health of the subject. In a preferred embodiment, it is an amount that is sufficient to effectively inhibit the calcineurin activity, thereby blocking or reducing the polyQ-huntingtin toxicity. The effect can be assessed by the phosphorylation state of huntingtin at position S421, through the neuronal death induced by polyQ-huntingtin. It will be appreciated that there will be many ways known in the art to determine the therapeutic amount for a given application. For instance, the dose of FK506 can be from 0.001 mg/kg/day to 10 mg/kg/day, preferably between 0.01 and 10 mg/kg/day by oral administration and between 0.001 and 1 mg/kg/day by intravenous injection. The administration protocols are well-known by one skilled in the art.

### EXAMPLES

The following example illustrates the invention.

### MATERIALS AND METHODS

***Constructs.*** The *pSIN-480-17*Q*, pSIN*-480-68Q, *pSIN-*480-68Q-S421A and *pSIN-*480-68QS421D were generated from the 480-17Q, 480-68Q, 480-68Q-S421A, and 480-68Q-S421D plasmids respectively (Humbert et al., 2002). These plasmids encode the first 480 amino acids fragment of huntingtin with 17 or 68 glutamines and a serine to alanine mutation (S421A) or a serine to aspartic acid mutation (S421D) at position 421. First, a PCR strategy was used to modify the C-terminal part of the 480 constructs (QuickChange site-directed mutagenesis; Stratagene, La Jolla, CA, USA) using the forward primer: 5' GCAGCTCACTCTGGTTCAAGAAGAG 3' (SEQ ID No 1) and the reverse primer :
5'CTCGAGTTAAGCGTAATCTGGAACATCGTATGGGTAGGATCTAGGC TGCTCAGTG 3' (SEQ ID No 2) containing a hemmaglutinin-tag (HA). The various fragments were cloned into the parental 480-17Q/68Q plasmids resulting in the generation of the vectors 480-17Q/68Q with S421, S421A and S421D mutation with a C-terminal HA tag and then subcloned in the SINW-PGK-GDNF *(BamHI-XhoI*; Deglon et al., 2000).

The lentiviral particles were produced in 293T cells and resuspended in phosphate-buffered saline (PBS)/1% bovine serum albumin (BSA) as previously reported). The particle content of viral batches was determined by p24 antigen ELISA (PerkinElmer Life Sciences, Boston, MA, USA).

The vectors encoding wild-type Akt, constitutively active Akt (Akt c.a.), wild-type CaNA, a constitutively-active (Ca2+-insensitive) form of CaNA (CaNA- ΔCaM), a catalytic-dead dominant interfering form of CaNA (CaNA-D130N) and CaNB, BDNF-eGFP, and the various huntingtin constructs 480-17Q, 480-68Q, 480-17Q-S421A, 480-68Q-S421A, 480-17Q-S421D, 480-68Q-S421D, WT and polyQ-FL-htt have been already described.

***Animals.*** Adult female Wistar rats (Iffa-Credo / Charles River, Les Oncins, France) weighting about 180-200 g were used. The animals were housed in a controlled temperature room that was maintained on a 12 h light/dark cycle. Food and water were available *ad libitum.*

FK506 (Alexis, Lausen, Switzerland) was administered to C57/BL6 male mice aged 5-6 weeks (Iffa-Credo/Charles River, Les Oncins, France) orally or by intraperitoneal injections (5 mg/kg) (Dunn et al., 1999; Singh et al., 2003). For oral administration, FK506 was dissolved in 100 µl 0.5% carboxymethylcellulose (Sigma). For intraperitoneal injections, FK506 was dissolved in 200 µl of Cremophor® (Sigma). Mice were sacrificed at indicated times post administration. The whole brain was homogenized in 1% Triton X-100 lysis buffer (see Western blot analysis section) supplemented with 1 µM okadaic acid (Sigma), 1 µM FK506 (Alexis), 1 µM cyclosporine A (Sigma) and 40 nM tautomycin (Calbiochem, Darmstadt, Germany) and centrifuged at 20,000 x g (5 min; 4°C). An aliquot of the supernatant was resolved by 6% SDS-PAGE.

Studies using animals were carried out in accordance with the Declaration of Helsinki and with the Guide for the Care and Use of Laboratory Animals, as adopted and promulgated by the National Institutes of Health, USA.

***Injection of the lentiviruses.*** Concentrated viral stocks were thawed and resuspended by repeated pipetting. Lentiviral vectors were stereotaxically injected (David Kopf Instruments, Tujunga, CA, USA) into the striatum of ketamine (75 mg/kg, i.p.) and xylazine (10 mg/kg, i.p.) anesthetized animals using an Hamilton syringe with a 34-gauge blunt-tip needle (Hamilton, Reno, NV, USA). For each vector, particle content was matched to 200,000 ng p24/ml. The viral suspensions (4 µl) were injected at 0.2 µl/min by means of an automatic injector (Stoelting Co., Wood Dale, IL, USA) and the needle was left in place for an additional 5 min. The stereotaxic coordinates were: 0.5 mm rostral to bregma; 3 mm lateral to midline and 5 mm from the skull surface. The skin was closed using a 6-0 Prolene® suture (Ethicon, Johnson and Johnson, Brussels, Belgium).

***Histological processing.*** 1, 12 and 24 weeks after lentiviral injection, the animals were given a sodium pentobarbital overdose and transcardially perfused with saline and 4% paraformaldehyde/10% picric acid. The brains were removed and postfixed in 4% paraformaldehyde/10% picric acid for approximately 24 h and finally cryoprotected in 25% sucrose/0.1 M phosphate buffer for 48 h. The brains were frozen in dry ice and 25 µm coronal sections were cut on a sliding microtome cryostat (Cryocut 1800, Leica Microsystems AG, Glattbrugg, Switzerland) at -20°C. Slices throughout the entire striatum were collected and stored in 48 well trays (Costar, Cambridge, MA, USA) as free floating sections in PBS supplemented with 0.12 µM sodium azide. The trays were stored at 4°C until immunohistochemical processing.

Striatal sections from injected rats were processed by immunochemistry for dopamine and cAMP-regulated phosphoprotein of a molecular mass of 32 kDa (DARPP-32; 1:7500; Chemicon International Inc., Temecula, CA, USA), and for HA-tag (monoclonal anti-HA antibody; 1:1000; Covance Research Products, Berkeley CA, USA) as previously described (Bensadoun et al., 2001). Sections were subsequently incubated with the biotinylated secondary goat anti-mouse or goat anti-rabbit antibodies (Vector laboratories, Burlingame, CA, USA) and the visualization was done as previously described

***Quantification of the DARPP-32 depleted regions and statistical analyses.*** The downregulation of DARPP-32 was measured with an image analysis program (NIH Image 1.63) in each section containing the lesion. For each section, the optical density is first determined in the targeted region, the striatum, to obtain the T value and, in a non infected region of the striatum (NI) that did not received the virus. A background value is then obtained from a DARPP-32-non expressing region (B, cortex). The percentage of lesion is then calculated using the following formula: % of lesion = 100 - ((NI - B) / (T - B) X 100) and is expressed as a percentage of lesion; 100% lesion corresponding to the lesion induced by the 480-68Q construct. Comparisons between two constructs injected in the same animal were made using paired t-tests. At 24 weeks, the striatal volume is not modified by the the various lentiviruses (data not shown).

***In vitro phosphorylation*/*dephosphorylation assays.*** Kinase assays were performed as described (Rangone et al., 2004) using recombinant Akt (Upstate Biotechnology, Charlottesville, USA) and a purified truncated form of huntingtin protein as a substrate (aminoacids 384 to 467 of human huntingtin fused with GST; 1 hr incubation, 30°C). Purified calcineurin (native protein isolated from bovine brain, Upstate Biotechnology, Temecula, CA, USA) was added as stated. The reaction products were resolved by 12 % SDS-PAGE.

***Cell culture, transfection and drug treatments.*** Primary cultures of striatal neurons were prepared from E17 Sprague Dawley rats and transfected at 4 days *in vitro* by a modified calcium phosphate technique (Saudou et al., 1998). Mouse neuronal cells derived from wildtype huntingtin mice (neuronal cells, +/+) and from Hdh^{Q109} knock-in mice (109Q/109Q) were cultured as previously described (Trettel et al., 2000) and transfected with Lipofectamine 2000 (Invitrogen, Breda, The Netherlands). When cotransfected (Fig. 3A), the ratio 480-17Q/Akt/CaNA-ΔCaM/CaNB was 1:0.5:1:1. Human HEK 293 cells were cultured in DMEM supplemented with 10 % bovine calf serum (BCS) and were transfected by the calcium phosphate technique. SHSY-5Y cells and Jurkat T cells were grown in RPMI supplemented with 10% BCS. Jurkat T cells were transferred to tyrodes buffer (137 mM NaCl, 3 mM KCl, 20 mM Hepes, 2 mM MgCl₂, 1 mM CaCl₂, 5.6 mM glucose, 0.3 mg/ml BSA; 4 x 10₆ cells/ml) prior addition of FK506 (1 µM) and/or ionomycin (2.5 µM).

***Western blot analysis.*** After transfection/incubation with drugs, cells were washed with icecold phosphate-buffered saline prior to scraping and lysis. Lysis buffer consisted on 50 mM Tris-HCl, pH 7.5, containing 0.1% Triton X-100, 2 mM EDTA, 2 mM EGTA, 50 mM NaF, 10 mM β-glycerophosphate, 5 mM sodium pyrophosphate, 1 mM sodium orthovanadate, 0.1% (v/v) β-mercaptoethanol, 250 µM PMSF, 10 mg/ml aprotinin and leupeptin. Cell lysates were centrifuged at 20,000 x g for 5 min at 4°C. Equal amounts of protein (40 µg) were subjected to SDS-PAGE and electrophoretically transferred to PVDF membranes (Immobilon-P, Millipore). Blots were blocked in 5% bovine serum albumin (BSA)/TBST buffer (20 mM Tris-HCl, 0.15 M NaCl, 0.1% Tween-20) and immunoblotted with anti-α- tubulin (1:1000; DM1A, Sigma), anticalcineurin Pan A (1:1000; Chemicon), anti-phosphohuntingtin- S421-763 (Humbert et al., 2002), anti-phospho-huntingtin-S421-714 (see below), anti-huntingtin 1259 (1:1000)) and MAB2166 (1:5000; Chemicon) antibodies for 1 hr. Blots were then labelled with anti-rabbit IgG:HRP (Jackson ImmunoResearch, West Grove, USA), washed and incubated for 5 min with SuperSignal WestPico Chemiluminescent Substrate (Pierce, Erembodegem, Belgium) according to the supplier's instructions. Membranes were exposed to Kodak Biomax films and developed. Quantification of the signal was performed by densitometric scaning of the film using ImageJ software.

***Immunofluorescence experiments.*** SHSY-5Y cells were grown on uncoated glass coverslips, transfected with wild-type CaNA/B for 24 hours and treated for 15 min with 1 µM ionomycin (Sigma) or left untreated. Cells were fixed with 4% paraformaldehyde-PHEM buffer for 20 min (in mM: PIPES, 120; HEPES, 50, EGTA, 20; Mg-acetate 4) and incubated with antiphospho- S421-huntingtin-763 (1:100) and anti-CaNA (1:200; Cat#C1956, Sigma) antibodies. Pictures were captured with a three-dimensional deconvolution imaging system as previously described (Gauthier et al., 2004). Mean fluorescence intensity of the phospho-S421 signal was next quantified using Metamorph software (Universal Imaging Corp, Princeton, NJ, USA) . For each pair of cells (transfected/non transfected), signal from the transfected cell was standardized by giving a value of 100 to the non-tranfected cell. Only non-mitotic cells of the same approximate size were considered. More than 30 cells in total were analyzed in two independent experiments.

Neurons were grown on laminin and polyD-lysine-coated glass coverslips, fixed as described before and incubated with the following primary antibodies: anti-phospho-S421-huntingtin- 714 (1:100), N-ter 1259 (1/500) and CaNA (1:200). Secondary antibodies were anti-mouse AlexaFluor-488 (1:200) and anti-rabbit AlexaFluor-555 (1:200; Molecular Probes, Eugene, OR, USA). Pictures were captured with a three-dimensional deconvolution imaging system.

***Measurement of neuronal survival.*** Four days post-plating, primary cultures of striatal neurons were transfected with wild-type or polyQ-huntingtin and green fluorescent protein (GFP) to identify the transfected cells. To be certain that each neuron synthesizing GFP also expressed the huntingtin construct, transfections were performed using a derived phosphate calcium method with a high ratio of huntingtin DNA to GFP DNA (10:1 ratio) (Humbert et al., 2002). Under these conditions over 95% of the GFP positive neurons also express the huntingtin construct (data not shown). GFP positive neurons were scored using fluorescence microscopy in a blinded manner 16 h and 36 h post transfection. Cell death occurring within the GFP positive cells was determined as the difference in the number of surviving neurons between the 2 time points and expressed as a fold increase in neuronal cell death relative to the death induced by the 480-17Q construct. Each graph represents two to four independent experiments performed in triplicate. Each bar in a given graph corresponds to the scoring of about 2000 neurons. For RNA interference experiment, neuronal cell death is expressed as the number of 480-68Q transfected cells that survived in presence of the various siRNA relative to the 480-68Q/scrambled condition. Data were submitted to complete statistical analysis.

***siRNA against calcineurin.*** The siRNA sequences targeting - rat CaNAα and CaNAβ correspond to the coding regions 677-695 (Acc. No. NM 017041) and 448-466 (Acc. No. NM 017042), respectively. More particularly, siRNA sequences targeting CaNAα are the following :
5' -CAGAGUAUUUCACGUUUAAdTdT- 3' (SEQ ID No 3)
3'-dTdTGUCUCAUAAAGUGCAAAUU- 5' (SEQ ID No 4)
and siRNA sequences targeting CaNAβ are the following :
5' -GGGUUGAUGUUCUGAAGAAdTdT- 3' (SEQ ID No 5)
3' -dTdTCCCAACUACAAGACUUCUU- 5' (SEQ ID No 6)
3 µg of siRNA or scrambled RNA were mixed with 4 x 10⁶ freshly isolated striatal neurons, nucleofected following manufacturer's instructions (Amaxa Biosystems, Germany), plated onto 12 well plates and incubated for 40 hours. The scrambled RNA has the same nucleotide composition than the CaNAα siRNA but lacks a significant sequence homology to any other gene.

***Antibodies against huntingtin phosphorylated on S421.*** Human specific anti-phosphohuntingtin- S421-763 antibody was previously described (Humbert et al., 2002). Generation of mouse specific anti-phospho-huntingtin-S421-714 antibody: phosphopeptide corresponding to mouse huntingtin sequence (CARGRSGS[PO₃H₂]IVELL) was synthesized, coupled to keyhole limpet hemocyanin (Neosystem, Strasbourg, France), and injected into rabbits. Polyclonal antibody was obtained from serum and affinity-purified with a phospho-peptide column. Briefly, the serum was filtered (0.22-µm filter), and following addition of 1 M Tris (pH 8.0) up to a final concentration of 100 mM, it was applied to a Sulfolink column (Pierce, Erembodegem, Belgium) coupled to the phosphorylated peptide. Retained antibodies were eluted with 100 mM glycine buffer (pH 2.7) and pH was rapidly neutralised with 1 M Tris pH 9. Antibodies were concentrated (Vivaspin concentrator 10000 MW, Viva Sience, Hannover, Germany) and stored in 50% glycerol.

***Videomicroscopy Experiments and Analyses.*** For videoexperiments rat primary cortical neurons, +/+, and 109Q/109Q cells established from *Hdh^{Q111}* knock-in mouse were prepared, cultured and transfected as previously described (Gauthier et al., 2004; Humbert et al., 2002; Saudou et al., 1998; Trettel et al., 2000). In some cases, primary cultures of cortical neurons are electroporated with the rat neuron Nucleofector® kit according to the supplier's manuel (Amaxa, Cologne). Forskolin (10 M; Sigma) and IBMX (100 M; Sigma) were added to the cultures 5 hours after transfection.

Videomicroscopy experiments were done two or four days after transfection. Cells were co-transfected with BDNF-eGFP and various constructs of htt or the corresponding empty vectors with a DNA ratio of 1:4. Live videomicroscopy was carried out using an imaging system previously detailed (Gauthier et al., 2004). Cells were grown on glass coverslip that was mounted in a Ludin chamber. The microscope and the chamber were kept at 37 °C (33 °C for knock-in cells). Stacks of 10-15 images with a Z-step of 0.3 µm were acquired with a 100X PlanApo N.A. 1.4 oil immersion objective coupled to a piezo device (PI). Images were collected in stream mode using a Cool Snap HQ camera (Ropper Scientific) set at 2 X 2 binning with an exposure time of 50 to 100 ms (frequency of 2). All stacks were treated by automatic batch deconvolution using the PSF of the optical system. Projections, animations and analyses were generated using ImageJ software. Dynamics were characterized by tracking positions of eGFP vesicles in cells as a function of time with a special develop plug-in (http://rsb.info.nih.gov/ij/plugins/track/track.html). During tracking, the Cartesian coordinates of the centers of vesicles were used to calculate dynamic parameters (velocity, pausing time, directionality).

### RESULTS

### Phosphorylation of polyQ-huntingtin at serine 421 is neuroprotective in vivo

The inventors have previously demonstrated that the IGF1/Akt pathway is neuroprotective in a cellular model of HD (Humbert et al., 2002). Indeed upon IGF-1 activation, Akt phosphorylates polyQ-huntingtin at S421 and blocks its toxicity in primary cultures of striatal neurons. To examine whether phosphorylation at S421 plays a role *in vivo* and could therefore be a therapeutic target, the inventors used a rat model of HD based on lentiviral-mediated expression of polyQ-huntingtin in the striatum. This model recapitulates several features observed in HD patients such as the presence of neuritic and intranuclear inclusions, neuronal dysfunction and death. The inventors generated HA-tagged lentiviral constructs encoding the first 480 amino acids of huntingtin containing 17 (wild-type, 480-17Q) or 68 glutamine residues (mutant, 480-68Q) with either an intact S421, a S421 to alanine (S421A) or a S421 to acid aspartic (S421D) mutation. Lentiviruses were then injected into rat striatum to allow direct comparison for a given rat between the 480-17Q/480-68Q, 480-68Q/480-68Q-S421A and 480-68Q/480-68Q-S421D constructs (Fig. 9). One week after injection, proper expression of the various huntingtin constructs was controlled by anti-huntingtin immunostaining and showed no differences in their expression levels (data not shown). According to an intermediate evaluation of the lesions at 12 weeks (data not shown), the inventors analyzed the polyQ-huntingtin-induced lesions in rat striatum 24 weeks after injection. Immunohistochemical analysis revealed that all constructs were expressed at similar levels and that expression of the transgenes were sustained at 24 weeks (Fig. 1). At this stage, no significant cell death was detected as indicated by the presence in the injected region of HA and Akt immunopositive neurons (Fig.1 and Fig. 10). Lesions were then evaluated using DARPP-32 as a marker. DARPP-32 is a regulator of the dopamine signaling that is present in about 96% of the striatal medium-sized spiny neurons. DARPP-32 is a marker of the dysfunction of these projecting neurons in HD as down-regulation of DARPP-32 is observed in various models of the disease. Immunohistochemical analysis with DARPP-32 antibody was performed and the size of the lesions was determined for all constructs and expressed as a percentage of the lesions induced by the 480-68Q constructs. As expected, the 480-68Q construct induced a significant lesion in contrast to wild-type huntingtin. The inventors found that absence of phosphorylation at S421 resulted in a strong increase (two-fold) in the size of the DARPP-32-depleted region induced by the 480-68Q construct. In contrast, constitutive phosphorylation at S421 led to a significant reduction (about 30 %) in the polyQ-induced DARPP-32-depleted region. Taken together, the inventors conclude that phosphorylation of S421 in huntingtin is crucial to regulate disease progression *in vivo.*

### Calcineurin dephosphorylates phospho-S421 of huntingtin in vitro

Because phosphatase activities could allow a dynamic regulation of S421 phosphorylation, the inventors aimed to identify phosphatases that would dephosphorylate S421. Calcineurin, also known as protein phosphatase 2B (PP2B), is a calcium- and calmodulin-dependent phosphatase (for reviews see Mansuy, 2003). To assess whether calcineurin acts on S421, the inventors first performed dephosphorylation experiments *in vitro.* For this purpose, they used a polyclonal antibody that only binds to the phospho-serine at S421 (anti-phospho-huntingtin-S421-763) on the huntingtin protein (Humbert et al., 2002). In addition, they have previously generated a GST-fused form of huntingtin (GST-huntingtin, amino acids 384 to 467 of human huntingtin fused to GST) that is phosphorylated by Akt and SGK on S421 (Humbert et al., 2002). They incubated this huntingtin fragment with a constitutive active form of Akt (Akt c.a.) resulting in the phosphorylation of GST-huntingtin at S421 as detected with the anti-phospho-huntingtin-S421-763 antibody (Fig. *2A, B*). This phosphorylated fragment of huntingtin was next incubated with purified calcineurin for different times (Fig. 2A) and for 60 min with different concentrations of calcineurin (Fig. 2*B*). As observed, calcineurin dephosphorylates S421 of huntingtin *in vitro* in a time and dose-dependent manner.

### Calcineurin dephosphorylates phospho-S421 of huntingtin in cells

Calcineurin is a heterodimer composed of a 60 kDa catalytic subunit (CaNA) and a 19 kDA regulatory subunit (CaNB) (Rusnak and Mertz, 2000). Heterodimeric holoenzyme is necessary for its phosphatase activity. To determine whether calcineurin dephosphorylation of huntingtin at S421 occurs in cells, the inventors cotransfected immortalized mouse striatal cells (+/+ cells) with 480-17Q, a constitutive active form of calcineurin A (CaNA-ΔCaM), CaNB and/or Akt (Fig. 3A). As expected, Akt induced the phosphorylation of huntingtin on S421 as detected by western-blotting with our newly generated anti-phospho-huntingtin-S421-714 antibody that recognizes preferentially the mouse sequence. The phosphorylation of S421 triggered by Akt was reduced by the cotransfection of active calcineurin (CaNA-ΔCaM/CaNB). Both calcineurin constructs were previously validated using a luciferase reporter of NFAT activity and induced a 15-fold NFAT activation when CaNA-ΔCaM/CaNB were cotransfected in HEK293 cells (not shown). Thus, the inventors demonstrate that calcineurin dephosphorylates the 480-17Q form of huntingtin at Akt-phosphorylated S421.

To test whether endogenous huntingtin is the target of calcineurin, the inventors used a dominant interfering form of CaNA where aspartic acid 130 is mutated into an asparagine (CaNA-D130N). Cotransfection of this construct with a NFAT reporter in HEK293 cells reduced endogenous NFAT activity by 60% (not shown). When CaNA-D130N encoding construct was transfected in the human neuroblastoma SHSY-5Y cell line, phosphorylation of endogenous huntingtin at S421 was increased (Fig. 3*B*). Therefore, phosphorylated S421 of endogenous huntingtin is a physiological substrate of calcineurin.

To further demonstrate that calcineurin indeed dephosphorylates huntingtin at S421 in cells, the inventors transfected neuronal cells with wild-type CaNA/CaNB and analyzed phosphorylation of endogenous huntingtin by immunofluorescence using the anti-phospho-huntingtin-S421-763 antibody in control conditions or after activation of calcineurin by the calcium ionophore ionomycin (Fig. 4*A*, higher panel). While huntingtin remained phosphorylated in the presence of calcineurin, they observed a reduction of huntingtin phosphorylation at S421 when calcineurin-transfected cells were treated with ionomycin (Fig. 4*A*, lower panel). Quantification of fluorescence intensity in more than 30 cells from two independent experiments revealed a statistically significant decrease in endogenous huntingtin phosphorylation at S421 (Fig. 4, graph). This demonstrates that activation of calcineurin by Ca²⁺ in neuronal cells leads to the dephosphorylation of S421 of huntingtin.

Finally, the inventors confirmed the relevance of their findings by verifying that in their experimental system, calcineurin and huntingtin are present in the same striatal neurons. They prepared primary cultures of striatal neurons that were subsequently immunostained for CaNA, huntingtin and phospho-S421-huntingtin. They found that most if not all calcineurin immunopositive striatal cells were also immunoreactive for total and phosphorylated huntingtin at S421 (Fig. 4*B*). Subcellularly, phosphohuntingtin and huntingtin were found in vesicular structures spread all along the cell body and the neurites. In addition to this prominent vesicular localization, huntingtin was also found in the cis-Golgi as demonstrated by the co-localization with GM130 (not shown). Interestingly, the inventors observed a partial co-localization of endogenous huntingtin and calcineurin that was particularly evident in vesicles along the neurites (see enlargements in Fig. 4*B*).

### A dominant-interfering form of calcineurin decreases -polyQ-huntingtin induced toxicity

The inventors have previously shown that phosphorylation of huntingtin at S421 is neuroprotective. They therefore investigated whether a dominant interfering form of calcineurin possesses neuroprotective properties by studying a neuronal model of HD that recapitulates the main features of the disease (Fig. 5*A*). They transfected primary cultures of striatal neurons with constructs 480-17Q and 480-68Q either alone or in the presence of CaNA-D130N, and analyzed neuronal death 24 h after transfection. As expected, the 480-68Q construct induced a statistically significant increase in neuronal death compared to the 480-17Q construct. Interestingly, transfection of CaNA-D130N decreased neuronal death induced by the 480-68Q fragment of huntingtin (Fig. 5*A*). These findings indicate that a dominant interfering form of calcineurin that increases phosphorylation of huntingtin on S421 exerts a neuroprotective effect on neuronal death induced by polyQ-huntingtin.

To further confirm the role of calcineurin inhibition on polyQ-huntingtin-induced cell death, the inventors decreased the levels of calcineurin by RNA interference. Two isoforms of calcineurin A subunits can be found in the brain, CaNAα and CaNAβ. The inventors therefore targeted both α and β subunits by RNA interference and found that the presence of both siRNA were necessary to ensure a significant decrease in the levels of CaNA (Fig. 5*B*). In these conditions, they observed a significant reduction in polyQ-huntingtin-induced cell death further supporting the role of calcineurin in this process.

Does the neuroprotection mediated by CaNA-D130N depend on S421? To answer this question, the inventors cotransfected CaNA-D130N in striatal neurons either with the 480-68Q construct or with a 480-68Q contruct where 5421 has been mutated into an alanine, 480-68Q-S421A (Fig. 5C). As previously mentioned, the CaNA-D130N construct protects neurons against polyQ-huntingtin induced toxicity, but it was unable to do so when position 421 could not be phosphorylated.

Therefore, calcineurin exerts its effect, at least in part, through the dephosphorylation of S421 on polyQ-huntingtin.

### Inhibition of calcineurin increases phosphorylation of huntingtin at S421 in cells

FK506 is an immunosuppressant drug used routinely in human treatment after transplantation. This compound mediates immunosuppression by inhibiting CaN-mediated dephosphorylation of NFAT, a transcription factor that regulates the expression of IL-2, which in turn regulates T-lymphocyte proliferation. Jurkat T cell line is a T-lymphocyte cell line extensively used to study calcium signaling pathways in which calcineurin participates (). To determine the effect of inhibiting calcineurin on the phosphorylation of huntingtin at S421, the inventors treated Jurkat T cells with FK506. As seen in figure 6A, FK506 increased the phosphorylation of S421 on endogenous huntingtin. The inventors next activated calcineurin by treating Jurkat T cells with the calcium ionophore ionomycin (Fig. 6*A*, lower panels). This resulted in a rapid dephosphorylation of huntingtin on S421, which was blocked by FK506.

The inventors next tested whether FK506 could also induce S421 phosphorylation in a cell type more relevant to the disease. They therefore treated primary cultured striatal neurons with FK506 for different times and concentrations (Fig. 6*B*). As for Jurkat cells, phosphorylation of endogenous huntingtin on S421 was increased over time and remained sustained for at least 6 hours. They also found that increasing concentrations of FK506 resulted in an increase in S421 phosphorylation. Together, these data show that inhibition of CaN activity with FK506 results in an increased phosphorylation of endogenous huntingtin on S421.

### PolyQ expansion leads to reduced huntingtin phosphorylation at S421 that can be increased by FK506: consequences on polyQ-induced neuronal toxicity

The inventors next assessed the level of phosphorylation of huntingtin in the pathological situation. They used mouse neuronal cells derived from knock-in mice in which a CAG expansion, encoding 109 glutamine residues, was inserted into the endogenous mouse huntingtin gene (109Q/109Q). This cell line closely resembles the situation in HD patients as, in these cells, polyQ-huntingtin is expressed at endogenous levels. The inventors performed immunoblotting experiments on +/+ or 109Q/109Q cells using our anti-phospho-huntingtin-S421-714 antibody and observed that phosphorylation of polyQ-huntingtin was drastically reduced compared to wild-type huntingtin (Fig. 7A). This agrees with previous work on transiently transfected HEK293 cells showing that S421 phosphorylation of polyQ-huntingtin was lower than of wild-type huntingtin (Warby et al., 2005). The inventors then asked whether inhibiting calcineurin would increase phosphorylation of polyQ-huntingtin on S421. Interestingly, treatment of 109Q/109Q cells with FK506 resulted in an increased S421 phosphorylation (Fig. 7B). The inventors have previously demonstrated that phosphorylation of huntingtin at S421 is neuroprotective. Therefore, as FK506 increases polyQ-huntingtin phosphorylation, they tested the effect of this immunosuppressive agent on polyQ-huntingtin-induced cell death (Fig. 7C). Primary cultures of striatal neurons were transfected with 480-68Q construct and treated with the vehicle or various doses of FK506. Treatment of cells with 0.3 and 1 µM of FK506 resulted in a decreased polyQ-induced neuronal death. Together, these data demonstrate that FK506, by preventing dephosphorylation of polyQ-huntingtin on S421, is neuroprotective in a cellular model of HD.

### A single administration of FK506 increases phosphorylation of huntingtin S421 in brain.

Since phosphorylation of huntingtin is crucial to regulating polyQ-huntingtin-induced toxicity *in vitro* and *in vivo* and considering that phosphorylation is reduced in disease models, the inventors aimed to determine whether FK506 administration could induce the phosphorylation of huntingtin *in vivo.* Mice were treated intraperitoneally or orally with FK506 and sacrificed at different times after administration. Brains were processed for immunoblotting and analyzed for huntingtin phosphorylation at S421 (Fig. 8). Strikingly, both intraperitoneal and oral administration of FK506 induced a sustained phosphorylation of endogenous htt on S421 in the brain (two/three-fold increase). In agreement with a specific effect of calcineurin on huntingtin phosphorylation, the inventors found, , that the level of Akt phosphorylation at S473 is not regulated by FK506. These results demonstrate that inhibition of calcineurin by administration of FK506 leads to the phosphorylation of endogenous huntingtin *in vivo.*

### IGF-1 and Akt rescue transport defect in HD

To test the possibility that the IGF-1/Akt pathway could regulate huntingtin-mediated transport in the context of HD, the inventors analyzed the dynamics of BDNF-containing vesicles using fast 3D videomicroscopy followed by deconvolution. Primary cultures of cortical neurons were transfected with BDNF-eGFP and either wild-type full length htt (wt-FL-htt, with 17Q), polyQ-FL-htt (with 75Q) or the corresponding vector. The inventors monitored the movement of BDNF-containing vesicles by acquiring images in 3D. After deconvolution, a 2D reconstruction of each time point was performed and individual vesicles were tracked to determine the two most relevant dynamic parameters of intracellular transport. The first parameter is the mean velocity of vesicles when they are moving (i.e., with speeds superior to 0) and the second correspond to the percentage of pausing time of vesicles (time spent by the vesicles without moving). As previouly reported, compared to wt-FL-htt, the presence of a polyQ expansion in htt results in a decrease in the mean velocity and an increase in the pausing time of BDNF-containing vesicles. Treatment of neurons with IGF-1 induced a statistically significant increase in the velocity of BDNF vesicles and decreased the pausing time of the vesicles (Figure 11A). It is worth to note that in presence of IGF-1, the dynamic parameters of intracellular transport by polyQ-FL-htt are indistinguishable from the wild-type situation suggesting an almost complete recovery of huntingtin function in transport by IGF-1 treatment.

The inventors next assessed the consequences of IGF-1 treatment in the HD genetic situation using neuronal cell lines derived from knock-in mice where a CAG expansion has been inserted into the endogenous mouse htt gene. These cell lines carry either two copies of wild-type htt (wild-type neuronal cells, +/+) or two copies of mutant htt (homozygous mutant neuronal cells, 109Q/109Q). These cell lines reflect the closest situation to HD patients as in these cells, wild-type or polyQ-htt are expressed at endogenous levels. While the velocity and pausing time of BDNF vesicles is significantly decreased in 109Q/109Q cells compared to +/+ cells (Figure 11B), the inventors found that IGF-1 treatment significantly restored vesicular transport by statistically increasing velocity and decreasing pausing time of BDNF-containing vesicles.

The inventors next tested whether Akt has a beneficial effect on intracellular transport in HD pathological situation. By cotransfection experiments of Akt and BDNF-eGFP, they found that Akt completely rescues intracellular transport in 109Q/109Q cells by increasing velocity and decreasing pausing time of BDNF-containing vesicles back to wild-type values (Figure 11C). Taken together, these findings indicate that the IGF-1/Akt pathway is able to rescue the alteration of intracellular transport of BDNF-containing vesicles that is observed in the HD pathological situation.

### Phosphorylation of S421 regulates huntingtin function in transport

The inventors next investigated the mechanisms by which IGF-1 and Akt rescue the defect of BDNF transport in HD mutant cells. Previously, they have shown that Akt phosphorylates huntingtin at S421 and that phosphorylation at this residue regulates polyQ-htt-induced toxicity in neuronal cells {Humbert, 2002}. In these experiments, they found that the neuronal toxicity induced by a N-terminal 480 amino acid fragment that contains a polyQ stretch is abrogated *in vitro* by phosphorylation at S421. Therefore, the inventors first investigated whether such N-terminal fragment is able to promote transport and whether it is affected by polyQ expansion. They analyzed in neuronal cells the ability of 480 fragments with a normal (17Q) or expanded polyQ stretch (68Q) and compared the dynamics of intracellular transport to those obtained with wt- and polyQ-FL-htt constructs. Interestingly, they found that wild type 480 fragment of htt is able to increase the velocity and to decrease the pausing time of BDNF-containing vesicles as efficiently as the full length fragment of wild type htt (Figure 12A). Morever they found that when such fragment contains the polyQ expansion, the transport is altered. They conclude that a N-terminal fragment of 480 amino acid of htt is able to promote transport and recapitulates the features observed with the full length htt on transport dynamics. In agreement, this fragment contains the HAP1-interacting region that corresponds to amino acids 171 to 231.

The inventors next analyzed the consequences of the absence of phosphorylation at S421 by using constructs that contain a serine to alanine mutation. Strikingly, they found that when wild type htt (480-17) is not phosphorylated at S421, it is no longer able to promote intracellular transport as demonstrated by the decrease in velocity and the increase in the percentage of pausing time (Figure 12B). Interestingly, the dynamic parameters obtained with the 480-17-S421A were not statistically significant from the dynamic parameters obtained with the constructs containing the polyQ expansion with a serine intact or mutated into an alanine, indicating that absence of phosphorylation at S421 turns htt into an inactive form for its capacity to enhance transport.

### Phosphorylation restores loss of huntingtin function

The alteration in the transport of BDNF in 109Q/109Q cells is rescued by Akt (Figure 11C). Therefore, the inventors investigated whether this effect is mediated by the phosphorylation of S421 by Akt and found that the ability of Akt to promote transport is lost when htt cannot be phosphorylated at S421 (Figure 12C). Having demonstrated that Akt rescues huntingtin-mediated transport through the phosphorylation of htt at S421, the inventors next tested the possibility that constitutive phosphorylation of S421 is sufficient to restore htt capacity to transport BDNF-containing vesicles. Strikingly they found that a polyQ-htt construct with a serine to aspartic acid mutation (480-68-S421D) to mimic constitutive phosphorylation competely recovers its capacity to transport BDNF-containing vesicles in contrast to the corresponding 480-68 construct as demonstrated by the increase velocity and decrease pausing time (Figure 12D).

Taken together, these results indicate that the functional activity of htt on transport is regulated by its phosphorylation at S421. In addition, the inventors demonstrate that the alteration of transport in disease situation can be restored by Akt through phosphorylation of S421 of htt.

### DISCUSSION

The inventors showed in the present invention that phosphorylation of huntingtin at S421 is crucial to regulating disease progression *in vivo.* Using a lentiviral approach to deliver N-terminal fragments of huntingtin directly to the striatum, they demonstrated that a construct encoding polyQ-huntingtin that is constitutively phosphorylated (480-68Q-S421D) induces a smaller DARPP-32-depleted region compared to a polyQ-huntingtin with an intact serine 421. Conversely, a polyQ-huntingtin that cannot be phosphorylated (480-68Q-S421A) is more toxic. The inventors have previously identified Akt and SGK as being able to act positively on S421 (Humbert et al., 2002; Rangone et al., 2004). They also reported that Akt is cleaved in post-mortem brain samples from HD patients (Humbert et al., 2002) and found that during disease progression, Akt activity is downregulated (Colin et al., 2005) further indicating that a reduced phosphorylation of huntingtin by Akt could occur in HD. In agreement, a decreased phosphorylation of polyQ-huntingtin at S421 is observed in YAC transgenic mice containing the polyQ expansion and in a cellular model of HD (Warby et al., 2005)(Fig. 7A). This implies that during disease progression, the phosphorylation of huntingtin at S421 diminishes, thereby increasing the toxicity of polyQ-huntingtin. Therefore, identifying the cellular mechanisms that control huntingtin phosphorylation at S421 could help to prevent excessive dephosphorylation of huntingtin during disease. In the present invention, the inventors demonstrated that calcineurin (CaN) is a phosphatase that targets huntingtin at S421. This indicates that calcineurin is a new cellular element that controls huntingtin phosphorylation at S421 and that could participate in HD pathogenesis.

The phosphorylation of a particular residue usually depends on a balance in the activities of tightly regulated kinases and phosphatases. The observed reduction in huntingtin phosphorylation could result from a reduction in Akt activity (Colin et al., 2005) but also from an excess of calcineurin phosphatase activity. Indeed, calcineurin phosphatase is highly expressed in the striatum, and in particular in the medium-size spiny neurons (MSNs), the first cells to degenerate in HD (Goto et al., 1989) and could therefore predispose huntingtin to dephosphorylation in these cells. Calcineurin is activated by Ca²⁺ (Mansuy, 2003) and several studies indicate that excessive Ca²⁺ entry in striatal neurons could play a role in HD. PolyQ-huntingtin facilitates the activity of type I inositol 1,4,5-triphosphate receptor (InsP₃R1) and NR2B subtype NMDA receptor, thereby leading to increased cytosolic Ca²⁺ levels. In MSNs the effect of mutant huntingtin on InsP₃R requires Huntingtin-associated protein-1, HAP1. As the connection between disturbed Ca²⁺ signalling and apoptosis is well established, it was proposed that glutamate released from corticostriatal projecting neurons elicits a supranormal Ca²⁺ response in MSNs from HD patients, leading to the opening of the mitochondria permeability transition pore (MPTP) and the activation of the caspase-dependent apoptotic cascade. In the present invention, the inventors propose that in addition to this mechanism, Ca²⁺, by activating calcineurin leads to dephosphorylation of huntingtin at S421 and participates in the pathogenic mechanism. In agreement, they showed that an increase in cytosolic Ca²⁺ concentration leads to dephosphorylation of huntingtin that can be blocked by FK506 demonstrating that the Ca²⁺-dependent dephosphorylation of S421 is dependent on CaN activity. Interestingly, another pathway by which calcineurin is activated in HD could involve calpain. Calpain is activated in HD. Calpain can regulate calcineurin activity either directly by cleaving calcineurin into an active form or by cleaving and inactivating Cabin1/cain, a potent endogenous inhibitor of calcineurin. Taken together, for the first time in HD, this study links excitotoxicity and Ca²⁺ to the phosphorylation of huntingtin.

The inventors' findings on the beneficial role of huntingtin phosphorylation at S421 on disease progression *in vivo* indicate that promoting huntingtin phosphorylation might have an impact on disease progression in HD patients as huntingtin phosphorylation is decreased when huntingtin contains the pathological polyQ expansion (Warby et al., 2005)(Fig. 7A). One strategy to increase huntingtin phosphorylation could involve drugs that activate the Akt or SGK pathway. However, hyperactivation of kinases such as Akt is associated with cancers (Franke et al., 2003) and drugs activating these pathways could have deleterious effects by inducing unwanted proliferative effects. Therefore, a more reasonable pharmacological approach to increase the phosphorylation of S421 is the inhibition of the phosphatase, once its identity is known. Many calcineurin inhibitors have been described of which cyclosporin A (CsA) and FK506 are the most potent, specific and well known.

In this study, the inventors focused on FK506 and found that FK506 is effective in inducing phosphorylation of huntingtin at S421 both *in vitro* and *in vivo* and in blocking polyQ-huntingtin-induced neuronal death. This suggests that FK506 is of therapeutic interest for HD patients.

As indicated previously, FK506 crosses the blood-brain barrier and, unlike CsA, does not require direct brain delivery of the compound. The fact that FK506 is routinely used in grafting procedures have established the safety and tolerability of such compounds and therefore could serve as a basis for similar therapeutic trials in HD.

### REFERENCES

Almeida S, Domingues A, Rodrigues L, Oliveira CR, Rego AC (2004) FK506 prevents mitochondrial-dependent apoptotic cell death induced by 3-nitropropionic acid in rat primary cortical cultures. Neurobiol Dis 17:435-444.
Bensadoun JC, de Almeida LP, Dreano M, Aebischer P, Deglon N (2001) Neuroprotective effect of interleukin-6 and IL6/IL6R chimera in the quinolinic acid rat model of Huntington's syndrome. Eur J Neurosci 14:1753-1761.
Cattaneo E, Rigamonti D, Goffredo D, Zuccato C, Squitieri F, Sipione S (2001) Loss of normal huntingtin function: new developments in Huntington's disease research. Trends Neurosci 24:182-188.
Charrin BC, Saudou F, Humbert S (2005) Axonal transport failure in neurodegenerative disorders: the case of Huntington's disease. Pathol Biol (Paris) 53:189-192.
Colin E, Regulier E, Perrin V, Durr A, Brice A, Aebischer P, Deglon N, Humbert S, Saudou F (2005) Akt is altered in an animal model of Huntington's disease and in patients. Eur J Neurosci 21:1478-1488.
Deglon N, Tseng JL, Bensadoun JC, Zurn AD, Arsenijevic Y, Pereira de Almeida L, Zufferey R, Trono D, Aebischer P (2000) Self-inactivating lentiviral vectors with enhanced transgene expression as potential gene transfer system in Parkinson's disease. Hum Gene Ther 11:179-190.
Dunn SE, Bums JL, Michel RN (1999) Calcineurin is required for skeletal muscle hypertrophy. J Biol Chem 274:21908-21912.
Franke TF, Hornik CP, Segev L, Shostak GA, Sugimoto C (2003) PI3K/Akt and apoptosis: size matters. Oncogene 22:8983-8998.
Gauthier LR, Charrin BC, Borrell-Pages M, Dompierre JP, Rangone H, Cordelieres FP, De Mey J, MacDonald ME, Lessmann V, Humbert S, Saudou F (2004) Huntingtin controls neurotrophic support and survival of neurons by enhancing BDNF vesicular transport along microtubules. Cell 118:127-138.
Goto S, Hirano A, Rojas-Corona RR (1989) An immunohistochemical investigation of the human neostriatum in Huntington's disease. Ann Neurol 25:298-304.
Hamawy MM (2003) Molecular actions of calcineurin inhibitors. Drug News Perspect 16:277-282.
Humbert S, Bryson EA, Cordelieres FP, Connors NC, Datta SR, Finkbeiner S, Greenberg ME, Saudou F (2002) The IGF-1/Akt pathway is neuroprotective in Huntington's disease and involves Huntingtin phosphorylation by Akt. Dev Cell 2:831-837.
Klettner A, Herdegen T (2003) FK506 and its analogs - therapeutic potential for neurological disorders. Curr Drug Targets CNS Neurol Disord 2:153-162.
Kremer B, et al. (1994) A Worldwide Study of the Huntington's Disease Mutation: The Sensitivity and Specificity of Measuring CAG Repeats. N. Engl. J. Med., 330, 1401-6.
Kurreck, J. (2003) Eur J Biochem. 270(8), 1628-44
Luo S, Vacher C, Davies JE, Rubinsztein DC (2005) Cdk5 phosphorylation of huntingtin reduces its cleavage by caspases: implications for mutant huntingtin toxicity. J Cell Biol 169:647-656.
Mansuy IM (2003) Calcineurin in memory and bidirectional plasticity. Biochem Biophys Res Commun 311:1195-1208.
Mittal, V. (2004) Nat Rev Genet 5(5): 355-65
Pong K, Zaleska MM (2003) Therapeutic implications for immunophilin ligands in the treatment of neurodegenerative diseases. Curr Drug Targets CNS Neurol Disord 2:349-356.
Rangone H, Poizat G, Troncoso J, Ross CA, MacDonald ME, Saudou F, Humbert S (2004) The serum- and glucocorticoid-induced kinase SGK inhibits mutant huntingtin-induced toxicity by phosphorylating serine 421 of huntingtin. Eur J Neurosci 19:273-279.
Ross CA (2002) Polyglutamine pathogenesis: emergence of unifying mechanisms for Huntington's disease and related disorders. Neuron 35:819-822.
Saudou F, Finkbeiner S, Devys D, Greenberg ME (1998) Huntingtin acts in the nucleus to induce apoptosis but death does not correlate with the formation of intranuclear inclusions. Cell 95:55-66.
Singh A, Kumar G, Naidu PS, Kulkarni SK (2003) Protective effect of FK506 (tacrolimus) in pentylenetetrazol-induced kindling in mice. Pharmacol Biochem Behav 75:853-860.
Usman, N., Blatt, L.M. (2000) J Clin Invest. 106(10), 1197-202
Warby SC, Chan EY, Metzler M, Gan L, Singaraja RR, Crocker SF, Robertson HA, Hayden MR (2005) Huntingtin phosphorylation on serine 421 is significantly reduced in the striatum and by polyglutamine expansion in vivo. Hum Mol Genet.

## Claims

1. Use of a drug increasing the phosphorylation of huntingtin at position S421 for the manufacture of a medicament for blocking or reducing the toxicity of polyQ-huntingtin in a subject having Huntington's disease.

2. Use according to claim 1, wherein the drug increasing the phosphorylation of huntingtin at position S421 is a drug increasing the activity of the kinase Akt, protein kinase A, Polo kinase 1, AuroraA, AuroraB and/or SGK.

3. Use according to claim 1, wherein the drug increasing the phosphorylation of huntingtin at position S421 is a drug inhibiting the dephosphorylation of huntingtin at position S421.

4. Use according to claim 3, wherein the drug inhibiting the dephosphorylation of huntingtin at position S421 is a calcineurin inhibitor or a drug inhibiting the interaction between calcineurin and huntingtin.

5. Use according to claim 4, wherein the calcineurin inhibitor is selected from the group consisting of cyclosporin A, FK506, FK520, L685,818, FK523, 15-0-DeMe-FK-520, Lie120, fenvalerate, resmethrin, cypermethrin, deltamethrin and an analogue thereof.

6. Use according to claim 5, wherein the calcineurin inhibitor is selected from the group consisting of FK506, cypermethrin, deltamethrin and an analogue thereof.

7. Use according to claim 6, wherein the calcineurin inhibitor is FK506.

8. Use according to claim 4, wherein the calcineurin inhibitor is a RNA interference specific of calcineurin.

9. Use according to claim 4, wherein the calcineurin inhibitor is a dominant-interfering form of calcineurin.

10. Use according any one of preceding claims, wherein the subject having Huntington's disease is presymptomatic.

11. Use according any one of claims 1-9, wherein the subject having Huntington's disease has symptoms of Huntington's disease.

12. Use according to claim 11, wherein said drug increasing the phosphorylation of huntingtin at position S421 is used in combination with a drug alleviating symptoms of Huntington's disease.

13. A method for selecting, identifying or screening a compound useful for treating a subject having Huntington's diseases, comprising the selection or identification of a compound capable of increasing the phosphorylation of huntingtin at position S421.

14. Method according to claim 13, wherein said method comprises:
a) providing a huntingtin protein or a fragment thereof of at least 50 consecutive amino acids comprising S421, said huntingtin protein or fragment thereof having a phosphorylated S421;
b) providing a calcineurin;
c) contacting a candidate compound with said huntingtin protein or fragment thereof and said calcineurin; and,
d) selecting the candidate compound that inhibits the dephosphorylation of huntingtin S421 by calcineurin.

15. Method according to claim 13, wherein said method comprises :
a) contacting a candidate compound with a cell expressing a huntingtin protein and comprising a kinase which phosphorylates huntingtin at position S421 and a calcineurin;
b) assessing the amount of huntingtin phosphorylated at position S421 and/or the amount of huntingtin which is not phosphorylated at position S421; and,
c) selecting the candidate compound that increases the phosphorylation of huntingtin at position S421 in comparison with a control cell which has not been contacted with the candidate compound.


SEQUENCE LISTING

<110> INSTITUT CURIE

<120> Method for treating Huntington's disease by inhibiting
dephosphorylation of huntingtin at S421

<130> B0435EP

<160> 6

<170> PatentIn version 3.1

<210> 1
<211> 25
<212> DNA
<213> artificial sequence

<220>
<223> primer
<400> 1
gcagctcact ctggttcaag aagag 25

<210> 2
<211> 55
<212> DNA
<213> artificial sequence

<220>
<223> primer
<400> 2
ctcgagttaa gcgtaatctg gaacatcgta tgggtaggat ctaggctgct cagtg 55

<210> 3
<211> 21
<212> DNA
<213> artificial sequence

<220>

<223>  siRNA

<400>  3
cagaguauuu cacguuuaat t                                                21

<210>  4

<211>  21

<212>  DNA

<213>  artificial sequence


<220>

<223>  siRNA

<400>  4
ttgucucaua aagugcaaau u                                                21

<210>  5

<211>  21

<212>  DNA

<213>  artificial sequence


<220>

<223>  siRNA

<400>  5
ggguugaugu ucugaagaat t                                                21

<210>  6

<211>  21

<212>  DNA

<213>  artificial sequence


<220>

<223>  siRNA

<400>  6
ttcccaacua caagacuucu u                                                21